(19) Europäisches Patentamt
European Patent Office
Office européen des brevets

(11) Veröffentlichungsnummer: **0 112 467**
**B1**

(12) # EUROPÄISCHE PATENTSCHRIFT

(45) Veröffentlichungstag der Patentschrift: **12.08.87**

(51) Int. Cl.⁴: **C 07 J 5/00**, A 61 K 31/57

(21) Anmeldenummer: **83110948.3**

(22) Anmeldetag: **03.11.83**

(54) **Neue 6alpha-Methylprednisolon-Derivate ihre Herstellung und Verwendung.**

(30) Priorität: **23.12.82 DE 3248435**

(43) Veröffentlichungstag der Anmeldung: **04.07.84 Patentblatt 84/27**

(45) Bekanntmachung des Hinweises auf die Patenterteilung: **12.08.87 Patentblatt 87/33**

(84) Benannte Vertragsstaaten: **AT BE CH DE FR GB IT LI LU NL SE**

(56) Entgegenhaltungen:
**FR-A-2 366 312**
**GB-A-1 026 160**

**STEROIDS, Band 7, Nr. 4, April 1966, Seiten 381-390, Holden Day, San Francisco, USA, M. HELLER et al.: "The preparation and biological activity of some 11beta-hydroxypregna-1,4,8-trienes"**

**Die Akte enthält technische Angaben, die nach dem Eingang der Anmeldung eingereicht wurden und die nicht in dieser Patentschrift enthalten sind.**

(73) Patentinhaber: **SCHERING AKTIENGESELLSCHAFT Berlin und Bergkamen, Müllerstrasse 170/178 Postfach 65 03 11, D-1000 Berlin 65 (DE)**

(72) Erfinder: **Annen, Klaus, Dr., Seegefelderstrasse 194, D-1000 Berlin 20 (DE)**
Erfinder: **Laurent, Henry, Dr., Glambecker Weg 21, D-1000 Berlin 28 (DE)**
Erfinder: **Hofmeister, Helmut, Dr., Weislingenstrasse 4, D-1000 Berlin 28 (DE)**
Erfinder: **Wiechert, Rudolf, Prof. Dr., Petzowerstrasse 8a, D-1000 Berlin 39 (DE)**
Erfinder: **Wendt, Hans, Dr., Ernst- Ring- Strasse 14, D-1000 Berlin 38 (DE)**

**EP 0 112 467 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die Erfindung betrifft neue 6α-Methylprednisolon-Derivate der allgemeinen Formel I

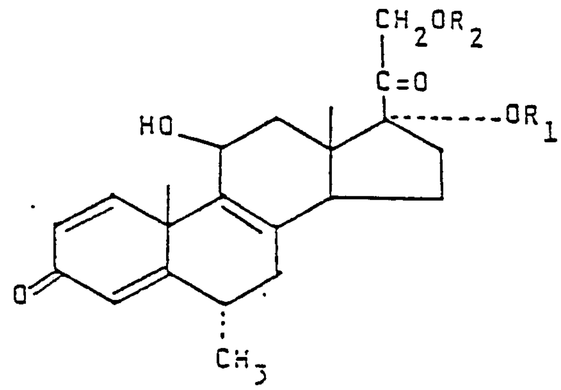

(I),

worin

$R_1$ einen 1-Oxoalkylrest mit 2 bis 6 Kohlenstoffatomen oder ein Benzoylrest und

$R_2$ ein Wasserstoffatom, einen 1-Oxoalkylrest mit 2 bis 6 Kohlenstoffatomen oder einen Benzoylrest darstellen, ein Verfahren zu ihrer Herstellung und pharmazeutische Präparate, die diese Verbindungen enthalten.

Die neuen 6α-Methylprednisolon-Derivate der allgemeinen Formel I gemäß Patentanspruch 1 können als 2 bis 6 Kohlenstoffatome enthaltende 1-Oxoalkylgruppen $R_1$ und $R_2$ beispielsweise eine Acetylgruppe, eine Propionylgruppe, eine Butyrylgruppe, eine Isobutyrylgruppe, eine Valerylgruppe, eine 3-Methylbutyrylgruppe, eine Trimethylacetylgruppe oder eine Hexanoylgruppe tragen.

Prednisolon-Derivate mit einer in der 8(9)-Position befindlichen Doppelbindung sind vorbekannt (Brit. Patentschrift 1,026,160 und die Publikation in "Steroids" 7, 1966, 381-390).

Die erfindungsgemäßen 6α-Methylprednisolon-Derivate zeigen bei topischer Applikation im bekannten Vasokonstriktionstest eine ausgeprägtere antiinflammatorische Wirksamkeit als diese vorbekannten strukturanalogen Verbindungen. Darüberhinaus zeichnen sie sich durch eine sehr gute Dissoziation zwischen erwünschter topischer Wirksamkeit und unerwünschter systemischer Wirkung aus, die größer ist als die der strukturanalogen vorbekannten Verbindungen.

Die neuen 6α-Methylprednisolon-Derivate können nach dem in dem Patentanspruch 7 gekennzeichneten Verfahren hergestellt werden, welches unter den Bedingungen durchgeführt werden kann, wie sie in den deutschen Patentanmeldungen Nr. 26 45 104, 26 45 105 und 23 40 591 und 19 58 549 sowie im US-Patent Nr. 3,383,394 oder in der Publikation J. Amer. Chem. Soc., 79, 1957, 1515 beschrieben sind. Die nachfolgenden Ausführungsbeispiele dienen zur Erläuterung des erfindungsgemäßen Verfahrens.

**Beispiel**

A) Eine Suspension von 34.0 g 21-Acetoxy-9α-brom-11β.17-dihydroxy-6α-methyl-1.4-pregnadien-3.20-dion in 1.36 l Methanol und 120 ml 70proz. Perchlorsäure wird 20 h bei Raumtemperatur gerührt. Nach der Eiswasserfällung wird der Niederschlag abgesaugt, mit Wasser neutralgewaschen und im Vakuumtrockenschrank getrocknet. Man erhält 28.3 g 9α-Brom-11β.17.21-trihydroxy-6α-methyl-1.4-pregnadien-3.20-dion. Schmp. 159-160°C.

B) Aus einer Lösung von 4.3 g 9α-Brom-11β.17.21-trihydroxy-6α-methyl-1.4-pregnadien-3.20-dion und 430 mg Pyridiniumtosylat in 34.5 ml Dimethylformamid und 300 ml Benzol werden bei 130°C über einen Wasserabscheider 129 ml Benzol abdestilliert. In die heiße Reaktionslösung läßt man 10.3 ml Orthobuttersäuretrimethylester hinzutropfen und destilliert anschließend weiter Benzol und andere leichtflüchtige Reaktionskomponenten ab. Man fügt 5 ml Pyridin hinzu und engt i.Vak. zur Trockne ein. Es wird 9α-Brom-11β-hydroxy-17α.21-(1-methoxybutylidendioxy)-6α-methyl-1.4-pregnadien-3.20-dion als Öl isoliert.

C) Das rohe 9α-Brom-11β-hydroxy-17α.21-(1-methoxybutyliden-dioxy)-6α-methyl-1.4-pregnadien-3.20-dion wird in 129 ml Methanol gelöst und mit einem Gemisch aus 46.4 ml 0.1N wäßriger Essigsäure und 5.2 ml 0.1M wäßriger Natriumacetat-Lösung 1 h bei 80°C Badtemperatur gerührt. Man engt die Lösung auf 1/3 ihres Volumens ein, gibt auf Wasser und wäscht die Essigesterextrakte neutral. Nach dem Trocknen und Einengen wird das Rohprodukt an 200 g Kieselgel mit einem Hexan-Aceton-Gradienten (0-60 % Aceton) gereinigt. Man isoliert 3.7 g 9α-Brom-17α-butyryloxy-11β.21-dihydroxy-6α-methyl-1.4-pregnadien-3.20-dion. Schmp. 158-159°C.

D) Eine Suspension von 3.0 g 9α-Brom-17α-butyryloxy-11β.21-dihydroxy-6α-methyl-1.4-pregnadien-3.20-dion in 60 ml Hexamethylphosphorsäuretriamid wird mit 3.0 g Lithiumchlorid 1 h bei 80°C Badtemperatur gerührt. Nach der Eiswasserfällung wird der Rückstand abfiltriert, mit Wasser gewaschen und das Rohprodukt an 105 g

Kieselgel mit einem Methylenchlorid-Aceton-Gradienten (0-20 % Aceton) gereinigt. Man isoliert 938 mg 17α-Butyryloxy-11β.21-dihydroxy-6α-methyl-1.4.8-pregnatrien-3.20-dion als Schaum. $[\alpha]^{25}_D$ = -53.8° (Chloroform).

**Beispiel 2**

A) Analog Beispiel 1B) werden 17.4 g 9α-Brom-11β.17α.21-trihydroxy-6α-methyl-1.4-pregnadien-3.20-dion mit 42.0 ml Orthobenzoesäuretriethylester umgesetzt und aufgearbeitet. Man isoliert 9α-Brom-17α.21-(1-ethoxybenzylidendioxy)-11β-hydroxy-6α-methyl-1.4-pregnadien-3.20-dion als Öl.

B) Das rohe 9α-Brom-17α.21-(1-ethoxybenzylidendioxy)-11β-hydroxy-6α-methyl-1.4-pregnadien-3.20-dion wird unter den Bedingungen des Beispiels 1C) hydrolysiert und aufgearbeitet. Das Rohprodukt wird an 1 kg Kieselgel mit einem Hexan-Aceton-Gradienten (0-50 % Aceton) gereinigt. Ausbeute 12.47 g 17α-Benzoyloxy-9α-brom-11β.21-dihydroxy-6α-methyl-1.4-pregnadien-3.20-dion. Schmp. 159° C.

C) Eine Lösung von 2.0 g 17α-Benzoyloxy-9α-brom-11β.21-dihydroxy-6α-methyl-1.4-pregnadien-3.20-dion wird mit 2.0 g Lithiumchlorid analog Beispiel 1D) umgesetzt und aufgearbeitet. Das Rohprodukt wird an 105 g Kieselgel mit einem Methylenchlorid-Aceton-Gradienten (0-20 % Aceton) gereinigt. Ausbeute 1.2 g 17α-Benzoyloxy-11β.21-dihydroxy-6α-methyl-1.4.8-pregnatrien-3.20-dion. Schmp. 206-208° C.

**Beispiel 3**

A) 3.0 g 17α-Benzoyloxy-9α-brom-11β.21-dihydroxy-6α-methyl-1.4-pregnadien-3.20-dion werden in 30 ml Pyridin mit 15 ml Acetanhydrid 3 h bei Raumtemperatur gerührt. Nach der üblichen Aufarbeitung erhält man 3.2 g 21-Acetoxy-17α-benzoyloxy-9α-brom-11β-hydroxy-6α-methyl-1.4-pregnadien-3.20-dion. Schmp. 172-173° C.

B) 3.3 g 21-Acetoxy-17α-benzoyloxy-9α-brom-11β-hydroxy-6α-methyl-1.4-pregnadien-3.20-dion werden analog Beispiel 1D) mit 3.3 g Lithiumchlorid umgesetzt und aufgearbeitet. Das Rohprodukt wird an 200 g Kieselgel mit einem Methylenchlorid-Aceton-Gradienten (0-15 % Aceton) gereinigt. Ausbeute 1.78 g 21-Acetoxy-17α-benzoyloxy-11β-hydroxy-6α-methyl-1.4.8-pregnatrien-3.20-dion. Schmp. 229-230° C.

**Beispiel 4**

A) Analog Beispiel 3A) werden 3.0 g 17α-Benzoyloxy-9α-brom-11β.21-dihydroxy-6α-methyl-1.4-pregnadien-3.20-dion mit Propionsäureanhydrid umgesetzt und aufgearbeitet. Man erhält 3.1 g 17α-Benzoyloxy-9α-brom-11β-hydroxy-6α-methyl-21-propionyloxy-1.4-pregnadien-3.20-dion. Schmp. 155-156° C.

B) 3.2 g 17α-Benzoyloxy-9α-brom-11β-hydroxy-6α-methyl-21-propionyloxy-1.4-pregnadien-3.20-dion werden unter den Bedingungen des Beispiels 1D) mit Lithiumchlorid umgesetzt, aufgearbeitet und gereinigt. Man isoliert 1.96 g 17α-Benzoyloxy-11β-hydroxy-6α-methyl-21-propionyloxy-1.4.8-pregnatrien-3.20-dion. Schmp. 225-226° C.

**Patentansprüche**

1. 6α-Methylprednisolon-Derivate der allgemeinen Formel I

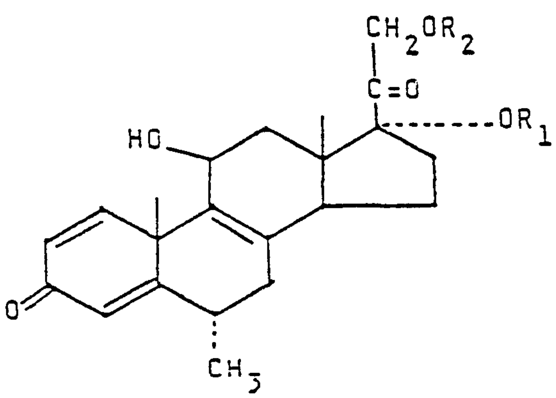

(I),

worin

$R_1$ einen 1-Oxoalkylrest mit 2 bis 6 Kohlenstoffatomen oder ein Benzoylrest und

$R_2$ ein Wasserstoffatom, einen l-Oxoalkylrest mit 2 bis 6 Kohlenstoffatomen oder einen Benzoylrest darstellen,

2. 17-Benzoyloxy-11β-Hydroxy-6α-methyl-1,4,8-pregnatrien-3,20-dion.

3. 17-Butyryloxy-11β,21-dihydroxy-6α-methyl-21-propionyloxy-1,4,8-pregnatrien-3,20-dion.

4. 21-Acetoxy-17-benzoyloxy-11β-hydroxy-6α-methyl-1,4,8-pregnatrien-3,20-dion.

5. 17-Benzoyloxy-11β,21-dihydroxy-6α-methyl-1,4,8-pregnatrien-3,20-dion.

6. Pharmazeutische Präparate gekennzeichnet durch den Gehalt an ein oder zwei 6α-Methylprednisolon-Derivaten gemäß Anspruch 1 bis 5.

7. Verfahren zur Herstellung von 6α-Methylprednisolon-Derivaten der allgemeinen Formel I

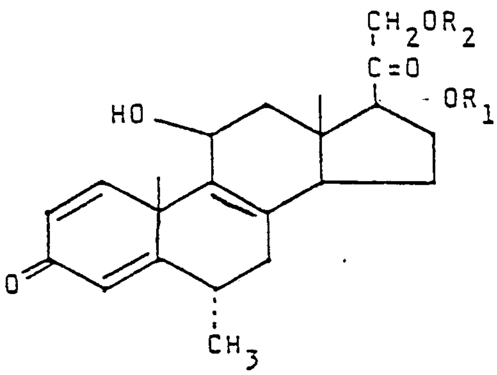

(I),

worin

$R_1$ einen 1-Oxoalkylrest mit 2 bis 6 Kohlenstoffatomen oder einen Benzoylrest und

$R_2$ ein Wasserstoffatom, einen 1-Oxoalkylrest mit 2 bis 6 Kohlenstoffatomen oder einen Benzoylrest bedeuten, dadurch gekennzeichnet, daß man in an sich bekannter Weise aus einem Kortikoid der allgemeinen Formel III

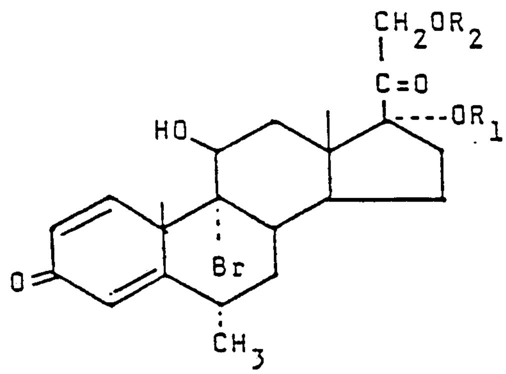

(III),

worin

$R_1$ und $R_2$ die obengenannte Bedeutung besitzen, Bromwasserstoff abspaltet.

## Claims

1. 6α-Methylprednisolone derivatives of the general formula I

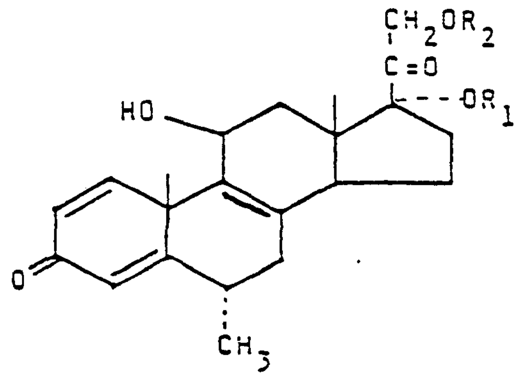

(I),

in which

$R_1$ represents a 1-oxoalkyl group of 2 to 6 carbon atoms or a benzoyl group and

$R_2$ represents a hydrogen atom, a 1-oxoalkyl group of 2 to 6 carbon atoms or a benzoyl group.

2. 17-Benzoyloxy-11β-hydroxy-6α-methyl-1,4,8-pregnatriene -3,20-dione.
3. 17-Butyryloxy-11β,21-dihydroxy-6α-methyl-21-propionyl-oxy-1,4,8-pregnatriene-3,20-dione.
4. 21-Acetoxy-17-benzyloxy-11β-hydroxy-6α-methyl-1,4,8-pregnatriene-3,20-dione.
5. 17-Benzoyloxy-11β,21-dihydroxy-6α-methyl-1,4,8-pregnatriene-3,20-dione.
6. Pharmaceutical preparations characterised in that they contain one or two 6α-methylprednisolone derivatives according to claims 1 to 5.
7. Process for the preparation of 6α-methylprednisolone derivatives of the general formula I

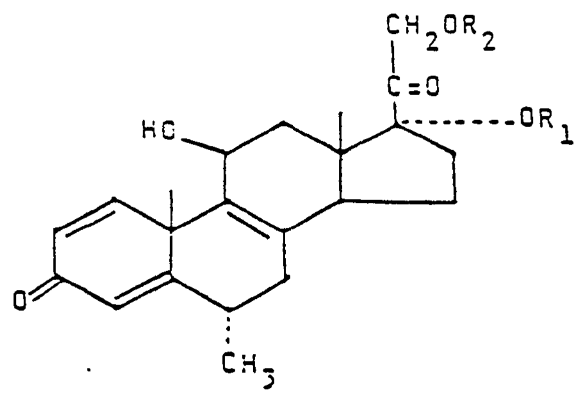

(I),

in which

$R_1$ represents a 1-oxoalkyl group of 2 to 6 carbon atoms or a benzoyl group and

$R_2$ represents a hydrogen atom, a 1-oxoalkyl group of 2 to 6 carbon atoms or a benzoyl group,

characterised in that hydrogen bromide is eliminated in a manner known per se from a corticoid of the general formula III

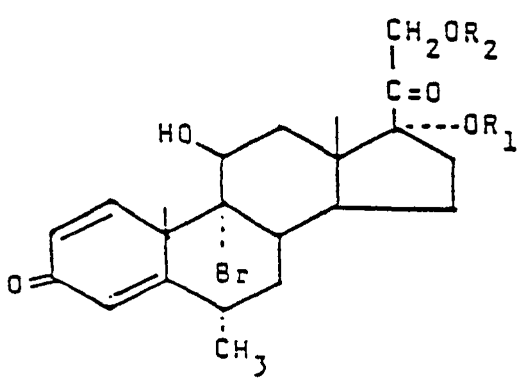

(III),

in which $R_1$ and $R_2$ have the meanings given above.

**Revendications**

1. Dérivés de la méthyl-6α prédnisolone qui répondent à la formule générale I:

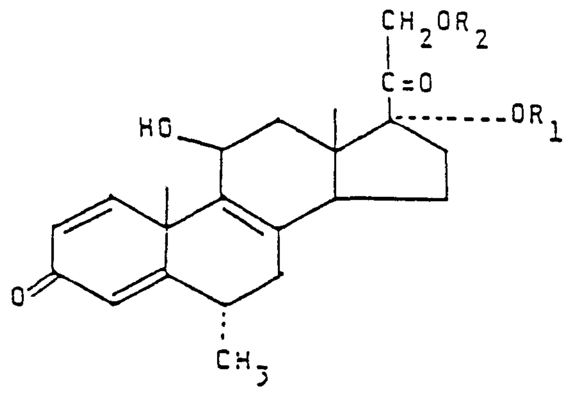

(I)

dans laquelle:

$R_1$ représente un radical oxo-1 alkyle contenant de 2 à 6 atomes de carbone ou un radical benzoyle et

$R_2$ représente un atome d'hydrogène, un radical oxo-1 alkyle contenant de 2 à 6 atomes de carbone ou un radical benzoyle.

2. Benzoyloxy-17 propionyloxy-21 hydroxy-11β méthyl-6α prégnatriène-1,4,8 dione-3,20.
3. Butyryloxy-17 dihydroxy-11β,21 méthyl-6α-prégnatriène-1,4,8 dione-3,20.
4. Acétoxy-21 benzoyloxy-17 hydroxy-11β méthyl-6α prégnatriène-1,4,8 dione-3,20.
5. Benzoyloxy-17 dihydroxy-11β,21 méthyl-6α prégnatriène-1,4,8 dione-3,20.
6. Compositions pharmaceutiques caractérisées en ce qu'elles contiennent un ou deux dérivés de la méthyl-6α prédnisolone selon l'une quelconque des revendications 1 à 5.
7. Procédé de préparation de dérivés de la méthyl-6α prednisolone de formule générale I:

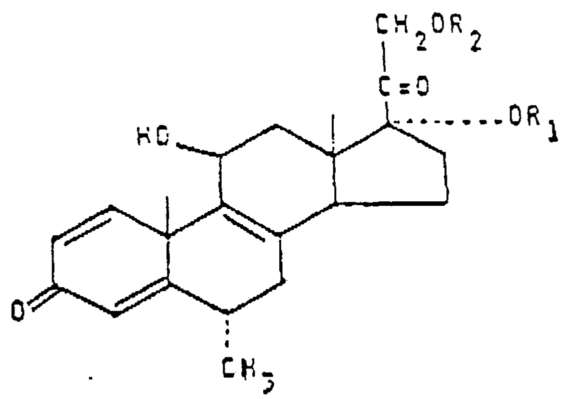

(I)

dans laquelle

$R_1$ représente un radical oxo-1 alkyle contenant de 2 à 6 atomes de carbone ou un radical benzoyle et

$R_2$ représente un atome d'hydrogène, un radical oxo-1 alkyle contenant de 2 à 6 atomes de carbone ou un radical benzoyle,

procédé caractérisé en ce qu'on élimine une mole de bromure d'hydrogène d'une mole d'un corticoide de formule générale III:

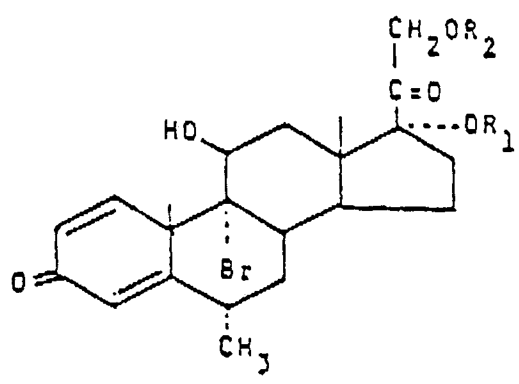

(III)

$R_1$ et $R_2$ ayant les significations indiquées ci-dessus.